# FASCICULE DE BREVET EUROPEEN

(11) **EP 1 378 230 B1**
(45) Date de publication et mention de la délivrance du brevet: **14.11.2007**
(21) Numéro de dépôt: 03291616.5
(22) Date de dépôt: 01.07.2003
(51) Int. Cl.: C07D 207/10, C07D 233/56, C07D 231/12, C07D 243/08, C07D 249/08, C07D 295/088, C07D 295/125, A61Q 5/10, A61K 8/49

(54) **Dérivés de para-phénylènediamine à groupement pyrrolidinyle substitué par un radical cyclique et leur utilisation pour la coloration de fibres kératiniques**
Para-Phenylendiaminderivate mit einer durch ein cyclisches Radikal substituierten Pyrrolidinylgruppe und ihre Verwendung zur Färbung von Keratinfasern
Para-phenylenediamine derivatives comprising a pyrrolidinyl group substituted by a cyclic radical and their use in colouring keratinic fibres

(30) Priorité: 05.07.2002 FR 0208514
(43) Date de publication de la demande: 07.01.2004
(73) Titulaire: L'ORÉAL, 75008 Paris (FR)
(72) Inventeur: Sabelle, Stéphane, 75005 Paris (FR); Ramos, Laure, 92340 Bourg Lareine (FR); Leduc, Madeleine, 75011 Paris (FR)
(74) Mandataire: Fevrier, Murielle Françoise E.

(56) Documents cités:
- WO-A-01/68043
- WO-A-02/45675

## Description

L'invention a pour objet de nouveaux dérivés de para-phénylènediamine à groupement pyrrolidinyle substitué par un radical particulier, les compositions tinctoriales les contenant ainsi que le procédé de teinture de fibres kératiniques à partir de ces compositions.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine, la para-toluène diamine sont des bases d'oxydation largement utilisées. Elles permettent d'obtenir avec des coupleurs d'oxydation des nuances variées.

Cependant, il existe un besoin de découvrir de nouvelles base d'oxydation présentant un meilleur profil toxicologique que la para-phénylènediamine et la para-toluène diamine, tout en permettant de conférer aux cheveux d'excellente propriétés d'intensité de couleur, de variété de nuances, d'uniformité de la couleur et de ténacité aux agents extérieurs.

Il est déjà connu d'utiliser des dérivés de para-phénylènediamine subtitués par un groupement pyrrolidinique comme base d'oxydation pour la coloration de fibres kératiniques. Par exemple, le brevet US 5,851,237 décrit l'utilisation de dérivés 1-(4-aminophényl)pyrrolidine éventuellement substitués sur le noyau benzénique afin de remplacer la para-phénylènediamine.

Le brevet US 5,993,491 propose l'utilisation de dérivés de N-(4-aminophényl)-2-hydroxyméthylpyrrolidine éventuellement substitués sur le noyau benzénique et sur l'hétérocycle pyrrolidinique en position 4 par un radical hydroxy afin de remplacer la para-phénylènediamine.

La demande de brevet JP 11-158048 propose des compositions contenant au moins un composé choisi parmi des dérivés de 4-aminoaniline éventuellement substitués sur le noyau benzénique et dont un des atomes d'azote est compris dans un cycle de 5 à 7 chaînons carbonés.

La demande de brevet EP 1 200 052 décrit des compositions tinctoriales contenant à titre de base d'oxydation des dérivés de para-phénylènediamine dont un des groupes amino forme un cycle pyrrolidinique substitué en position 3 par un groupe amino.

Ces composés ne permettent pas de conférer aux cheveux une coloration de qualité équivalente à celle obtenue avec la para-phénylènediamine ou avec la para-toluènediamine du fait d'un manque d'intensité et d'uniformité de la couleur.

Il existe donc un réel besoin de découvrir de nouvelles bases d'oxydation présentant à la fois un bon profil toxicologique et des propriétés telles que les compositions les contenant permettent de conférer aux cheveux d'excellentes propriétés d'intensité de couleur, de variété de nuances, d'uniformité de la couleur et de ténacité vis à vis des différentes agressions extérieures que peuvent subir les cheveux.

Le but de la présente invention est de développer de nouvelles compositions tinctoriales ne présentant pas les inconvénients des bases d'oxydation de la technique antérieure en fournissant de nouvelles compositions tinctoriales pour la teinture de fibres kératiniques qui ne dégradent pas les fibres kératiniques, tout en étant capables d'engendrer des colorations intenses dans des nuances variées, peu sélectives particulièrement résistantes et présentant un bon profil toxicologique.

Ce but est atteint avec la présente invention qui a pour objet des dérivés de para-phénylènediamine substitués par un groupement pyrrolidinyle de formule (I) et leurs sels d'addition 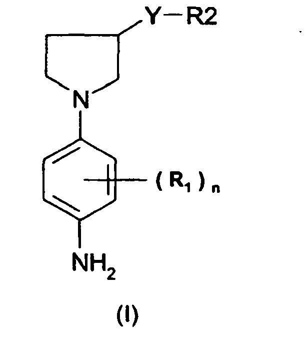 dans laquelle
- n est compris entre 0 et 4, étant entendu que lorsque n est supérieur ou égal à 2 alors les radicaux R₁ peuvent être identiques ou différents,
- R₁ représente un atome d'halogène; une chaîne hydrocarbonée en C₁-C₆, aliphatique ou alicyclique, saturée ou insaturée, un ou plusieurs atomes de carbone de la chaîne hydrocarbonée pouvant être remplacée par un ou plusieurs atomes d'oxygène, d'azote, de silicium, de soufre, par un groupement SO ou SO₂, le radical R₁ ne comportant pas de liaison peroxyde, ni de radicaux diazo, nitro ou nitroso ; cette chaîne pouvant être substituée par un ou plusieurs atomes d'halogène, un ou plusieurs radicaux hydroxyle, amino, mono- ou di-alkyl(C₁-C₄)amino, tri-alkyl(C₁-C₄)ammonium,
- Y représente une liaison covalente ou une chaîne alkylène en C₁-C₁₄, linéaire ou ramifiée, un ou plusieurs atomes de carbone de cette chaîne pouvant être remplacés par un atome d'oxygène, d'azote, de soufre, SO ou SO₂ ; la chaîne pouvant être substituée par un radical hydroxyle, alcoxy en C₁-C₆ ,amino, alkylamino en C₁-C₆ ou dialkylamino en C₁-C₆ ; la chaîne pouvant porter une ou plusieurs fonctions cétones,
- R₂ représente un cycle imidazolique.

L'invention a aussi pour objet une composition tinctoriale contenant au moins un dérivé de para-phénylènediamine de formule (I) à titre de base d'oxydation.

Un autre objet de l'invention est l'utilisation de ces dérivés pour la teinture de fibres kératiniques ainsi qu'un procédé de teinture de fibres kératiniques, en particuliers les fibres kératiniques humaines telles que les cheveux mettant en oeuvre de la composition de la présente invention.

La composition de la présente invention permet en particulier d'obtenir une coloration de fibres kératiniques chromatique, puissante, peu sélective, tenace et qui présente un bon profil toxicologique.

Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne ramifiée contenant une structure cyclique et pouvant contenir une ou plusieurs insaturations du type alcène ou alcyne mais ne contenant pas de structure cyclique aromatique.

Lorsque les atomes de carbone de la chaîne sont remplacés par un atome T d'oxygène, de soufre, d'azote, de silicium, SO ou SO₂, on obtient par exemple pour T un motif-T-CH₂-, -T-.

A titre d'exemple, R₁ peut être un atome de chlore ou de brome, un radical méthyle, éthyle, isopropyle, vinyle, allyle, méthoxyméthyle, hydroxyéthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1-amino-2-hydroxyéthyle, 1,2-diaminoéthyle, méthoxy, éthoxy, allyloxy, 2-hydroxyéthyloxy.

Dans le cadre de l'invention, n est de préférence égal à 0 ou 1.

Selon un mode de réalisation particulier, R₁ est choisi parmi un atome d'halogène, un radical alkyle en C₁-C₄, un radical hydroxyalkyle en C₁-C₄, un radical aminoalkyle en C₁-C₄, un radical alcoxy en C₁-C₄, un radical hydroxyalcoxy en C₁-C₄. A titre d'exemple, R₁ est choisi parmi un radical méthyle, hydroxyméthyle, 2-hydroxyéthyle, 1,2-dihydroxyéthyle, méthoxy, isopropyloxy, 2-hydroxyéthoxy.

Lorsque Y représente une liaison covalente alors R₂ est directement relié au cycle pyrrolidinique. Lorsque Y représente une chaîne alkylène, alors Y est de préférence une chaîne alkylène en C₁-C₈ pouvant contenir un ou plusieurs motifs choisis parmi -O-, -NR'- ou -NR'CO- avec R' représentant un hydrogène ou un radical alkyle en C₁-C₄. Y peut aussi représenter les motifs suivants -O-, -NR'-, -S-, -SO- ou -SO₂.

Selon un mode de réalisation particulier, R₂ est relié à Y par l'intermédiaire d'un des atomes d'azote de l'hétérocycle azoté.

Les composés de formule (I) peuvent être sous forme de sels d'acide avec des acides minéraux forts tels que par exemple HCI, HBr, H₂SO₄, ou avec des acides organiques tels que, par exemple, l'acide acétique, lactique, tartrique, citrique ou succinique.

A titre d'exemples de dérivés de formule (I), on peut citer :

| | | | |
|---|---|---|---|
| 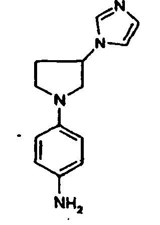 | 4-(3-lmidazol-1-yl-pyrrolidin-1-yl)-phénylamine | | |
| 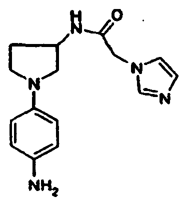 | N-[1-(4-Amino-phényl)-pyrrolidin-3-yl]-2-imidazol-1-yl-acétamide | 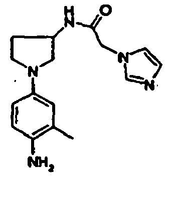 | N-[1-(4-Amino-3-méthylphényl)-pyrrolidin-3-yl]-2-Imidazol-1-yl-acétamide |
| 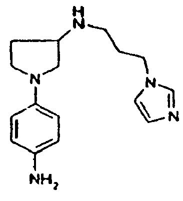 | [1-(4-Amino-phényl)-pyrrolidin-3-yl]-(3-imidazol-1-yl-propyl)-amine | 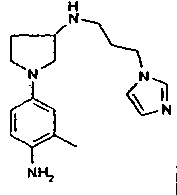 | 2-Méthyl-[1-(4-Aminophényl)-pyrrolidin-3-yl]-(3-imidazol-1 -yl-propyl)-amine |
| 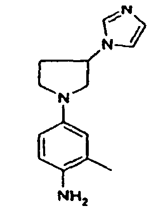 | 4-(3-lmidazol-1-yl-pyrrolidin-1-yl)-2-méthyl-phénylamine | | |

Parmi ces composés, les composés suivants sont particulièrement préférés :
-4-(3-Imidazol-1-yl-pyrrrolidin-1-yl)-phénylamine
[1-(4-Amino-phényl)-pyrrolidin-3-yl]-(3-imidazol-1-yl-propyl)-amine
-2-Méthyl-[1-(4-Amino-phényl)-pyrrolidin-3-yl]-(3-imidazol-1-yl-propyl)-amine
-4-(3-Imidazol-1-yl-pyrrolidin-1-yl)-2-méthyl-phénylamine

La composition tinctoriale de la présente invention comprend, dans un milieu cosmétiquement acceptable et approprié pour la teinture des fibres kératiniques, en particulier les fibres kératiniques humaines, à titre de base d'oxydation au moins un dérivé de formule (I) tel que défini précédemment.

La ou les bases d'oxydation de l'invention sont en général présentent chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthytamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylénedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation. A titre d'exemple, ces bases d'oxydation additionnelles sont choisies parmi les para-phénylènediamines autres que celles décrites précédemment, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(p-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthytoxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine; la 2-acétylamino pyrazolo-[1,5-a] pyridin-3-ylamine ; la 2-morpholin-4-yl-pyrazolo[1,5-a]pyridin-3-ylamine; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol; le 2-(3-amino-pyrazolo[1,5-a]pyridine-5-yl)-éthanol; le 2-(3-amino-pyrazolo[1,5-a]pyridine-7-yl)-éthanol ; le (3-amino-pyrazolo[1,5-a]pyridine-2-yl)-méthanol; la 3,6-diamino-pyrazolo[1,5-a]pyridine ; la 3,4-diamino-pyrazolo[1,5-a]pyridine; la pyrazolo[1,5-a]pyridine-3,7-diamine ; la 7-morpholin-4-yl-pyrazolo[1,5-a]pyridin-3-ylamine; la pyrazolo[1,5-a]pyridine-3,5-diamine; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol; 3-amino-pyrazolo[1,5-a]pyridine-4-ol; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571; JP 05-63124; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 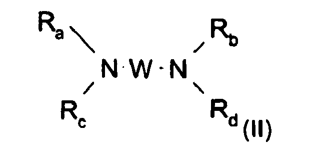 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour obtenir la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### EXEMPLES DE SYNTHESE

### Exemple 1 : synthèse de la 4-(3-imidazolyl-1yl-pyrrolidin-1yl)phénylamine

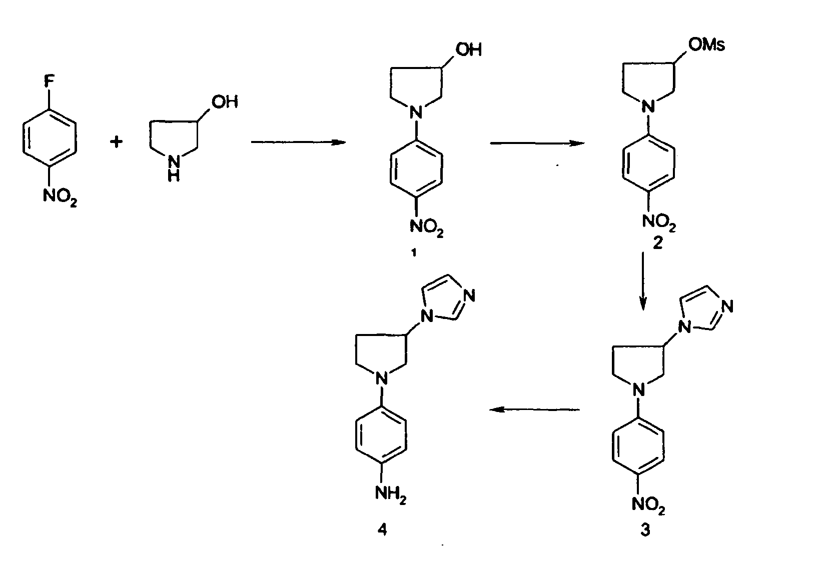

### I. Synthèse de l'ester d'acide méthanesulfonique et de 1-(4-nitro-phényl)-Pyrrolidin-3yl (2)

A 83,3g (0,4mole) de N-(4-nitrophényl)-3-hydroxypyrrolidine en solution dans 625ml de THF anhydre et 72,7ml (0,6mole) de triéthylamine, on ajoute goutte à goutte 40ml (0,516mole) de chlorure de mésyle à 5°C. On laisse revenir à température ambiante, puis on verse dans la glace pour former un précipité.
Après essorage et séchage du précipité, on obtient 109 g de poudre jaune (2).
**Point de fusion** = 203°C
**RMN 1H** (400MHz-DMSO) ppm 8.09(d, 2 H); 6.68(d, 2H) ;5.47(m,1H) ;3.77-3.48 (m,4H) ; 3.28(s,3H); 2.35(m,2H).

### II Synthèse du 1-[1-(4-nitro-phényl)-pyrrolidin-3-yl]-1H-imidazole (3)

22g (0,0767mole) d'ester d'acide méthanesulfonique et de 1-(4-nitro-phényl)-pyrrolidin-3yl **(2)** sont chauffés 2 heures à 95°C dans 170g d'imidazole (2,5 mole), Ce mélange est versé dans 1l d'eau glacée jusqu'à cristallisation. Après filtration et séchage, on chromatographie la poudre jaune obtenue, éluant dichlorométhane et on récupère 14,5g de dérivé (**3**) (rendement 73,2%)
**point de fusion** =163°C
**RMN 1H** (400MHz-DMSO) ppm 8.20(m, 2H); 7.87 (s, 1 H); 7.38(s, 1H); 7.06(s, 1H); 6.81(m, 2H) ;5.17(m,1 H); 4.06(m, 1H); de 3.79 à 3.65 (m,3H); 2.66(m,1H) ; 2.50(m,1H);
**Masse ESI+** : m/z=259 [M+]

### III. Synthèse de la 4-(3-imidazolyl-1yl-pyrrolidin-1yl)phénylamine; chlorhydrate (4)

13,5g (0,0522 mole) du dérivé précédent en solution dans 700ml d'éthanol sont hydrogénés en présence de palladium sur charbon sous une pression d'hydrogène de 8 bars. Après filtration du catalyseur, le dérivé (**4**) attendu est isolé sous forme de chlorhydrate. On obtient 13g de poudre blanche, rendement 82,7%.
**RMN 1H** (400MHz-DMSO) ppm 9.29(s, 1H); 7.84(t, 1 H); 7.71(t, 1H); 7.26(m, 2H); 6.67(m, 2H); 5.29(m,1H) ; 3.77(m, 1H) ; 3.68 (m, 2H); 3.38(m, 1H); 2.51 (m,1H); 2.46 (m, 1H);
Masse ESI+ : m/z=229 [M+]

### Exemple 2 : synthèse du 4-(-[1,3']bipyrrolinyl-1'-yl)phénylamine

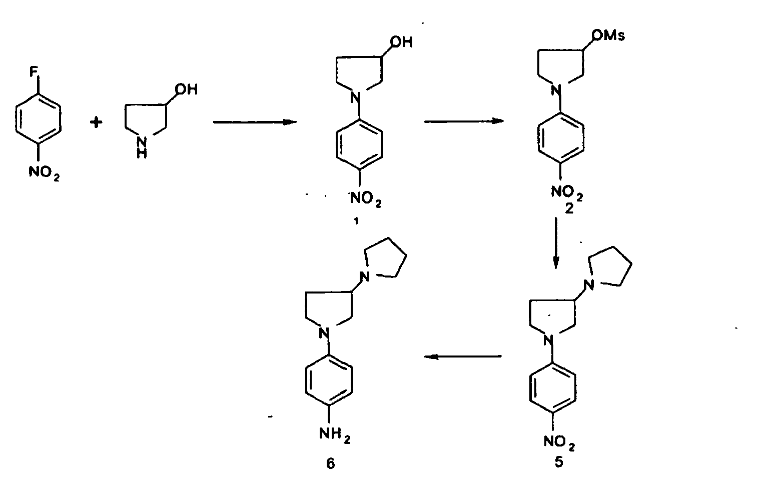

### I. Synthèse du 1'-(4-nitro-phenyl)-[1,3]bipyrrolidinyl (5)

5 g (0.0174 mole) de l'ester de l'acide méthanesulfonique et du 1-(4-nitro-phenyl)-pyrrolidin-3yl **(2)** sont chauffés 2 heures à 85°C dans 30 ml de pyrrolidine (mole). Ce mélange est versé dans de l'eau glacée jusqu'à cristallisation. Après filtration et séchage, on chromatographie la poudre jaune obtenue, éluant dichlorométhane/méthanol (98/2) et récupère 2,6 g de dérivé (**5**) .(rendement 53 %)
**point de fusion**=114°C
**RMN 1H** (400MHz-DMSO) ppm 8.04(m, 2H) ; 6.61(m, 2 H) ; 3.60(m, 2H); 3.40(m, 1H); 3.24(m, 1H); 2.86(m,1H); 2.50(m, 2H); 2.16(m, 1H); 1.92(m, 1H); 1.70(m, 4H).
**Masse ESI⁺** : m/z=262 [M+]

### II. Synthèse de la 4-(-[1,3']bipyrrolinyl-1'-yl)phenylamine: chlorhydrate (6)

2,5 g (0,0096 mole) du dérivé précédent **(5)** en solution dans 400ml d'éthanol sont hydrogénés en présence de palladium sur charbon sous une pression d'hydrogène de 8 bars à une température de 50°C; après filtration du catalyseur, le dérivé (**6**) attendu est isolé sous forme de chlorhydrate. On obtient 1.3g de poudre blanche, rendement 44%.
**RMN 1H** (400MHz-D20) ppm 7.33(m, 2H); 6.86(m, 2H); 4.11(m,1H); 3.75(m, 3H); 3.60 (m, 2H); 3.39(m, 1H); 2.60 (m,1 H); 2.31(m, 1H); 2.18(m, 2H), 2.06(m, 2H).
**Masse ESI+** : m/z=232 [M+]

### Exemple 3 : synthèse de la [1-(4-Amino-phényl)-pyrrolidin-3-yl]-(3-imidazol-1-yl-propyl)-amine

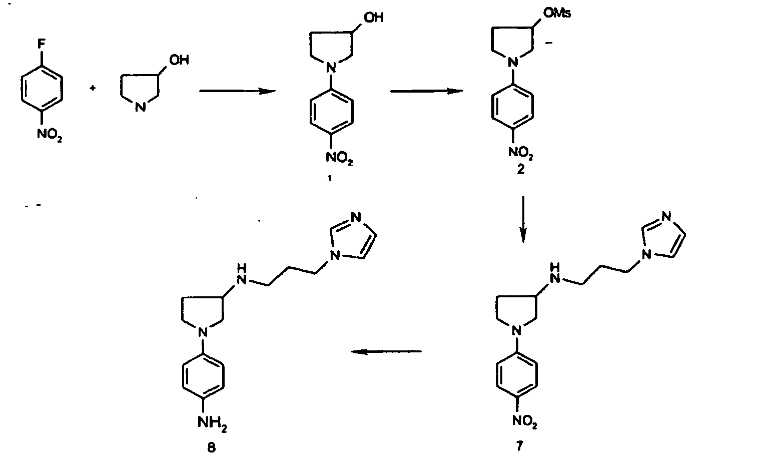

### I. Synthèse de la [3-(Imidazol-1-yl)-propyl]-[1-(4-nitro-phényl)-pyrrolidin-3-yl]-amine (7)

30 g (0,105 mole) de l'ester d'acide méthane sulfonique et de 1-(4-nitro-phenyl)-pyrrolidin-3yl **(2)** sont chauffés 18 heures à 90°C avec 120 g d'aminopropylimidazole (0,958 mole). Ce mélange est versé dans de l'eau glacée et le produit est extrait au dichlorométhane. On chromatographie la poudre jaune obtenue, éluant dichlorométhane/ méthanol (98/2) et récupère 15,2 g de dérivé (**7**) .(rendement 48,2 %)
**Point de fusion**=**74°C**
**RMN 1H** (400MHz-DMSO) ppm 8.06(m, 2H) ; 7.60(s, 1 H) ; 7.15(s, 1H); 6.84(s, 1H); 6.61(m, 2H) ; 4.35(bs,1H) ; 4.03(m, 2H) ; 3.(m,2H) ; 3.49(m,2H) ; 3.24(m,1H) ; 2.15(m, 1 H) ; 1.85(m, 3H).
**Masse ESI+** : m/z=316 [M+]

### II. Synthèse du [1-(4-Amino-phényl)-pyrrolidin-3-yl]-(3-imidazol-1-yl-propyl)-amine; chlorhydrate (8)

6.5g (0.0206mole) du dérivé précédent (**7**) en solution dans 500ml d'éthanol sont hydrogénés en présence de palladium sur charbon sous 10bars; après filtration du catalyseur, le dérivé (**8**) attendu est isolé sous forme de chlorhydrate. On obtient 7.07g de poudre blanche, rendement 86%.
**RMN 1H** (400MHz-D20) ppm 8.86(s, 1H); 7.63(d, 1 H); 7.55(d, 1H); 7.34(d, 2H); 6.83(d, 2H); 4.46(t,1H); 4.13(m, 1H); 3.72(m, 1H); 3.61(m, 2H); 3.40(m,1H) ; 2.58(m, 1H) ; 2.43(m, 1H); 2.31(m,1H).
**Masse ESI+** : m/z=286 [M+]

### EXEMPLES DE TEINTURE

### EXEMPLES 1 A 11 : TEINTURE EN MILIEU ALCALIN

| **Exemples** | **1** | **2** | **3** | **4** | **5** | **6** | **7** | **8** | **9** | **10** | **11** |
|---|---|---|---|---|---|---|---|---|---|---|---|
| 4-(3-Imidazol-1-yl-pyrrolidin-1-yl) phenylamine (base) | 10⁻³ | 10⁻³ | 10⁻³ | 10⁻³ | - | - | - | - | - | - | - |
| [1-(4-Amino-phenyl)pyrrolidin-3-yl]-(3-imidazol-1-yl-propyl)-amine (base) | - | - | - | - | 10⁻³ | 10⁻³ | 10⁻³ | - | - | - | - |
| 4-[1,3']Bipyrrolidinyl-1'-yl-phenylamine (base) | - | - | - | - | - | - | - | 10⁻³ | 10⁻³ | 10⁻³ | 10⁻³ |
| 2-(2,4-Diamino-phénoxy)-éthanol, dichlorhydrate (coupleur) | 10⁻³ | - | - | - | 10⁻³ | - | - | 10⁻³ | - | - | - |
| 3-Amino-2-chloro-6-méthyl-phénol, chlorhydrate (coupleur) | - | 10⁻³ | - | - | - | - | - | - | 10⁻³ | - | - |
| 3.6-Diméthyl-1H-pyrazolo[5,1-c] [1.2.4]triazole (coupleur) | - | - | 10⁻³ | - | - | 10⁻³ | - | - | - | 10⁻³ | - |
| 2-méthyl-5-aminophénol (coupleur) | - | - | - | 10⁻³ | - | - | 10⁻³ | - | - | - | 10⁻³ |
| Support de teinture (1) | (*) | (*) | (*) | (*) | (*) | (*) | (*) | (*) | (*) | (*) | (*) |
| Eau déminéralisée q.s.p. | 100g | 100g | 100g | 100g | 100g | 100g | 100g | 100g | 100g | 100g | 100g |

| | | | | | | | | | | | |
|---|---|---|---|---|---|---|---|---|---|---|---|
| - les quantités de base et de coupleur sont exprimées en mole (*) Support de teinture (1) pH 9,5 | | | | | | | | | | | |

| | |
|---|---|
| Alcool éthylique à 96° | 20,8 g |
| Métabisulfite de sodium en solution aqueuse à 35% | 0,23g M.A |
| Sel pentasodique de l'acide diéthylène triamine-pentaacétique en solution aqueuse à 40 % | 0,48g M.A |
| Alkyl en C₈-C₁₀polyglucoside en solution aqueuse à 60% | 3,6g M.A |
| Alcool benzylique | 2,0g |
| Polyéthylène glycol à 8 motifs d'oxyde d'éthylène | 3,0g |
| NH₄Cl | 4,32g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

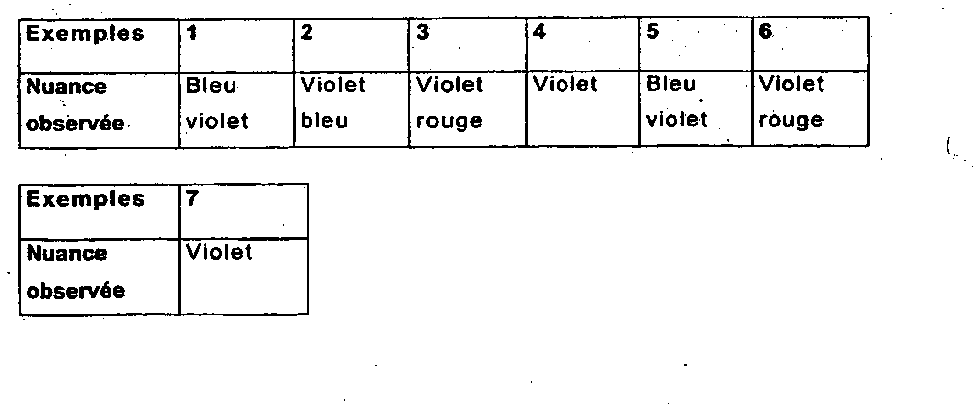

### EXEMPLES 12 A 29 : TEINTURE EN MILIEU ACIDE

On a préparé les compositions tinctoriales suivantes :

| **Exemples** | **12** | **13** | **14** | **15** | **16** | **17** | **18** | **19** | **20** | **21** | **22** | **23** | **24** | **25** | **26** | **27** | **28** | **29** |
|---|---|---|---|---|---|---|---|---|---|---|---|---|---|---|---|---|---|---|
| 4-(3-Imidazol-1-yl-pyrrolidin-1-yl)-phénylamine (base) | 10⁻³ | 10⁻³ | 10⁻³ | 10⁻³ | 10⁻³ | 10⁻³ | - | - | - | - | - | - | - | - | - | - | - | - |
| [1-(4-Amino-phényl)-pyrrolidin-3-yl]-(3- imidazol-1-yl-propyl)-amine (base) | - | - | - | - | - | - | 10⁻³ | 10⁻³ | 10⁻³ | 10³ | 10⁻³ | 10⁻³ | - | - | - | - | - | - |
| 2-(2,4-Diaminophénoxy)-éthanol. dichlorhydrale (coupleur) | 10⁻³ | - | - | - | - | - | 10⁻³ | - | - | - | - | - | 10⁻³ | - | - | - | - | - |
| 3-Amino-2-chloro-6-méthyl-phénol, chlorhydrate (coupleur) | - | 10⁻³ | - | - | - | - | - | 10⁻³ | - | - | - | - | - | 10⁻³ | - | - | - | - |
| 2-méthyt-5-aminophénol (Coupleur) | - | - | 10⁻³ | - | - | - | - | - | 10⁻³ | - | - | - | - | - | 10⁻³ | - | - | - |
| 2-Amino-pyridin-3-ol (coupleur) | - | - | - | 10⁻³ | - | - | - | - | - | 10⁻³ | - | - | - | - | - | 10⁻³ | - | - |
| 3,6-Diméthyl-1H-pyrazolo[5.1-c] [1,2.4]triazole (coupleur) | - | - | - | - | 10⁻³ | - | - | - | - | - | 10⁻³ | - | - | - | - | - | 10⁻³ | - |
| 6-Hydroxy-1-H-indole (coupleur) | - | - | - | - | - | 10⁻³ | - | - | - | | -- | 10⁻³ | - | - | - | - | - | 10⁻³ |
| Support de teinture (2) | (*) | | | | | | | | | | | | | | | | | |
| Eau déminéralisée q.s.p. | 100g | | | | | | | | | | | | | | | | | |

| | | | | | | | | | | | | | | | | | | |
|---|---|---|---|---|---|---|---|---|---|---|---|---|---|---|---|---|---|---|
| - Les quantités de base et de coupleur sont exprimées en mole (*) Support de teinture (2) pH 7 | | | | | | | | | | | | | | | | | | |

| | |
|---|---|
| Alcool éthylique à 96° | 20 g |
| Métabisulfite de sodium en solution aqueuse à 35% | 0,23g M.A |
| Sel pentasodique de l'acide diéthylène tria mine pentaacétique en solution aqueuse à 40 % | 0.48g M.A |
| Alkyl en C₈-C₁₀polyglucoside en solution aqueuse à 60% | 3,6g M.A |
| Alcool benzylique | 2,0g |
| Polyéthylène glycol à 8 motifs d'oxyde d'éthylène | 3,0 g |
| Na₂HPO₄ | 0,28 g |
| KH₂PO₄ | 0.46 g |

Au moment de l'emploi, chaque composition est mélangée avec un poids égal d'eau oxygénée à 20 volumes (6% en poids). On obtient un pH final de 7 .

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

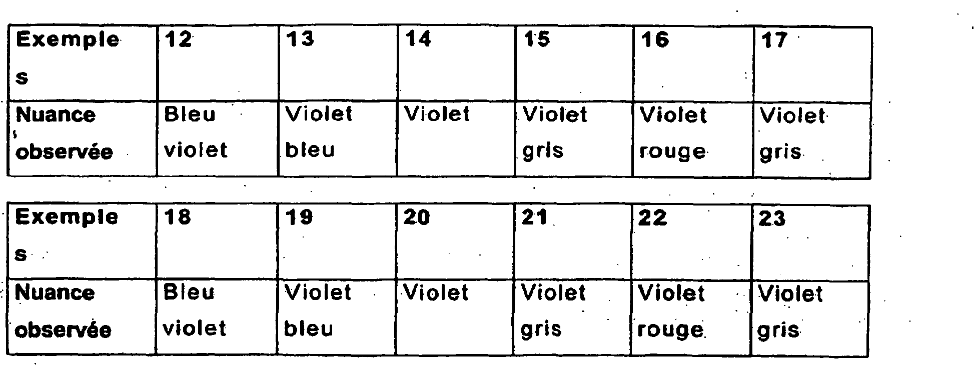

## Revendications

1. Dérivés de para-phénylènediamine substitués par un groupement pyrrolidinyle de formule (I) et leurs sels d'addition 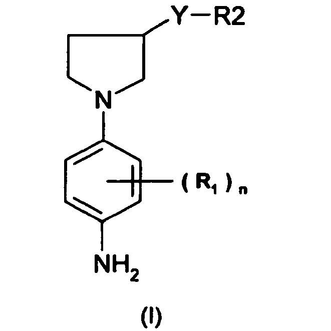 dans laquelle
• n est compris entre 0 et 4, étant entendu que lorsque n est supérieur ou égal à 2 alors les radicaux R₁ peuvent être identiques ou différents,
• R₁ représente un atome d'halogène ; une chaîne hydrocarbonée en C₁-C₆, aliphatique ou alicyclique, saturée ou insaturée, un ou plusieurs atomes de carbone de la chaîne hydrocarbonée pouvant être remplacée par un ou plusieurs atomes d'oxygène, d'azote, de silicium, de soufre, par un groupement SO ou SO₂, le radical R₁ ne comportant pas de liaison peroxyde, ni de radicaux diazo, nitro ou nitroso ; cette chaîne pouvant être substituée par un ou plusieurs atomes d'halogène, un ou plusieurs radicaux hydroxyle, amino, mono- ou di-alkyl(C₁-C₄)amino, tri-alkyl(C₁-C₄)ammonium,
• Y représente une liaison covalente ou une chaîne alkylène en C₁-C₁₄, linéaire ou ramifiée, un ou plusieurs atomes de carbone de cette chaîne pouvant être remplacés par un atome d'oxygène, d'azote, de soufre, SO ou SO₂ ; la chaîne pouvant être substituée par un radical hydroxyle, alcoxy en C₁-C₆ ,amino, alkylamino en C₁-C₆ ou dialkylamino en C₁-C₆ ; la chaîne pouvant porter une ou plusieurs fonctions cétones,
• R₂ représente un cycle imidazolique.

2. Dérivés selon la revendication 1 dans lesquels n est égal à 0 ou 1.

3. Dérivés selon la revendication 1 ou 2 dans lesquels R₁ est un atome d'halogène, un radical alkyle en C₁-C₄, un radical hydroxyalkyle en C₁-C₄, un radical aminoalkyle en C₁-C₄, un radical alcoxy en C₁-C₄, un radical hydroxyalcoxy en C₁-C₄.

4. Dérivés selon la revendication 1 ou 3 dans lesquels R₁ est choisi parmi un radical méthyle, hydroxyméthyle, 2-hydroxyéthyle, 1,2-dihydroxyéthyle, méthoxy, isopropyloxy, 2-hydroxyéthoxy.

5. Dérivés selon l'une quelconque des revendications 1 à 4 dans lesquels Y est une liaison covalente.

6. Dérivés selon l'une quelconque des revendications 1 à 5 dans lesquels Y est une chaîne alkylène en C₁-C₈ pouvant contenir un ou plusieurs motifs choisis parmi -O-, -NR'- ou -NR'CO- avec R' représentant un hydrogène ou un radical alkyle en C₁-C₄.

7. Dérivés selon l'une quelconque des revendications 1 à 6 dans lesquels Y représente -0-, -NR'-, -S-, -SO- ou -SO₂-.

8. Dérivés selon la revendication 8 ou 9 dans lesquels R₂ est relié à Y par l'intermédiaire d'un des atomes d'azote du cycle imidazolique.

9. Dérivés selon l'une quelconque des revendications 1 à 8 choisis parmi la 4-(3-Imidazol-1-yl-pyrrolidin-1-yl)-phénylamine, la [1-(4-Amino-phényl)-pyrrolidin-3-yl]-(3-imidazol-1-yl-propyl)-amine, la 2-Méthyl-[1-(4-Amino-phényl)-pyrrolidin-3-yl]-(3-imidazol-1-yl-propyl)-amine, la 4-(3-Imidazol-1-yl-pyrrolidin-1-yl)-2-méthyl-phénylamine.

10. Dérivés selon la revendication 9 choisis parmi la 4-(3-Imidazol-1-yl-pyrrolidin-1-yl)-phénylamine, la [1-(4-Amino-phényl)-pyrrolidin-3-yl]-(3-imidazol-1-yl-propyl)-amine, la 2-Méthyl-[1-(4-Amino-phényl)-pyrrolidin-3-yl]-(3-imidazol-1-yl-propyl)-amine, la 4-(3-lmidazol-1-yl-pyrrolidin-1-yl)-2-méthyl-phénylamine,

11. Composition tinctoriale comprenant à titre de base d'oxydation au moins un dérivé para-phénylènediamine de formule (I), tel que défini selon l'une quelconque des revendications 1 à 10.

12. Composition selon la revendication 11 comprenant de plus un coupleur choisi parmi les métaphénylènediamines, les méta-aminophénols, les métadiphénols, les coupleurs naphtaléniques, les coupleurs hétérocycliques et leurs sels d'addition

13. Composition selon l'une quelconque des revendications 11 ou 12 comprenant une base d'oxydation additionnelle autre que les bases d'oxydation de formule (I) choisie parmi les para-phénylènediamines, les bis-phénylalkylènediamines, les para-aminophénols, les ortho-aminophénols, les bases hétérocycliques et leurs sels d'addition.

14. Composition selon l'une quelconque des revendications précédentes 11 à 13 dans laquelle la quantité de chacune des bases d'oxydation est comprise entre 0,001 à 10 % en poids environ du poids total de la composition tinctoriale.

15. Composition selon la revendication 12 dans laquelle la quantité de chacun des coupleurs est comprise entre 0,001 et 10 % en poids environ du poids total de la composition tinctoriale.

16. Composition selon l'une quelconque des revendications 11 à 15 comprenant un milieu cosmétiquement acceptable approprié pour la teinture des fibres kératiniques.

17. Composition selon l'une quelconque des revendications 11 à 16 comprenant un agent oxydant.

18. Procédé de teinture d'oxydation des fibres kératiniques, **caractérisé en ce qu'**on applique sur les fibres une composition tinctoriale telle que définie dans l'une quelconque des revendications 11 à 16 en en présence d'un agent oxydant pendant un temps suffisant pour développer la coloration désirée.

19. Procédé selon la revendication 18 dans lequel l'agent oxydant est choisi parmi le peroxyde d'hydrogène, le peroxyde d'urée, les bromates de métaux alcalins, les persels, les peracides et les enzymes oxydases.

20. Dispositif à plusieurs compartiments dans lequel un premier compartiment contient une composition tinctoriale telle que définie à l'une quelconque des revendications 11 à 16 et un deuxième compartiment contient un agent oxydant.

21. Utilisation de la composition définie aux revendications 11 à 17 pour la teinture de fibres kératiniques.

## Claims

1. para-Phenylenediamine derivatives substituted with a pyrrolidinyl group of formula (I), and the addition salts thereof 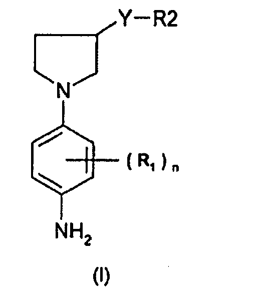 in which
• n is between 0 and 4, it being understood that when n is greater than or equal to 2, then the radicals R₁ may be identical or different,
• R₁ represents a halogen atom; a C₁-C₆ aliphatic or alicyclic, saturated or unsaturated hydrocarbon-based chain, one or more carbon atoms of the hydrocarbon-based chain possibly being replaced with one or more oxygen, nitrogen, silicon or sulfur atoms or with an SO or SO₂ group, the radical R₁ not comprising a peroxide bond or diazo, nitro or nitroso radicals; this chain possibly being substituted with one or more halogen atoms or one or more hydroxyl, amino, mono- or di (C₁-C₄) alkylamino or tri (C₁-C₄)alkylammonium radicals,
• Y represents a covalent bond or a linear or branched C₁-C₁₄ alkylene chain, one or more carbon atoms of this chain possibly being replaced with an oxygen, nitrogen or sulfur atom or with SO or SO₂; the chain possibly being substituted with a hydroxyl, C₁-C₆ alkoxy, amino, C₁-C₆ alkylamino or C₁-C₆ dialkylamino radical; the chain possibly bearing one or more ketone functions,
• R₂ represents an imidazole ring.

2. Derivatives according to Claim 1, in which n is equal to 0 or 1.

3. Derivatives according to Claim 1 or 2, in which R₁ is a halogen atom, a C₁-C₄ alkyl radical, a C₁-C₄ hydroxyalkyl radical, a C₁-C₄ aminoalkyl radical, a C₁-C₄ alkoxy radical or a C₁-C₄ hydroxyalkoxy radical.

4. Derivatives according to Claim 1 or 3, in which R₁ is chosen from methyl, hydroxymethyl, 2-hydroxyethyl, 1,2-dihydroxyethyl, methoxy, isopropyloxy and 2-hydroxyethoxy radicals.

5. Derivatives according to any one of Claims 1 to 4, in which Y is a covalent bond.

6. Derivatives according to any one of Claims 1 to 5, in which Y is a C₁-C₈ alkylene chain possibly containing one or more units chosen from -O-, -NR'- and -NR' CO- with R' representing a hydrogen atom or a C₁-C₄ alkyl radical.

7. Derivatives according to any one of Claims 1 to 6, in which Y represents -O-, -NR'-, -S-, -SO- or -SO₂-.

8. Derivatives according to Claim 8 or 9, in which R₂ is linked to Y via one of the nitrogen atoms of the imidazole ring.

9. Derivatives according to any one of Claims 1 to 8, chosen from 4-(3-imidazol-1-ylpyrrolidin-1-yl)phenylamine, [1-(4-aminophenyl)pyrrolidin-3-yl](3-imidazol-1-ylpropyl)amine, 2-methyl[1-(4-aminophenyl)pyrrolidin-3-yl](3-imidazol-1-ylpropyl)amine and 4-(3-imidazol-1-yl-pyrrolidin-1-yl)-2-methylphenylamine.

10. Derivatives according to Claim 9, chosen from 4-(3-imidazol-1-ylpyrrolidin-1-yl)phenylamine, [1-(4-aminophenyl)pyrrolidin-3-yl](3-imidazol-1-ylpropyl)-amine, 2-methyl[1-(4-aminophenyl)pyrrolidin-3-yl](3-imidazol-1-ylpropyl)amine and 4-(3-imidazol-1-ylpyrrolidin-1-yl)-2-methylphenylamine.

11. Dye composition comprising, as oxidation base, at least one para-phenylenediamine derivative of formula (I), as defined according to any one of Claims 1 to 10.

12. Composition according to Claim 11, also comprising a coupler chosen from meta-phenylenediamines, meta-aminophenols, meta-diphenols, naphthalene-based couplers and heterocyclic couplers, and the addition salts thereof.

13. Composition according to either of Claims 11 and 12, comprising an additional oxidation base other than the oxidation bases of formula (I), chosen from para-phenylenediamines, bis(phenyl)alkylenediamines, para-aminophenols, ortho-aminophenols and heterocyclic bases, and the addition salts thereof.

14. Composition according to any one of the preceding Claims 11 to 13, in which the amount of each of the oxidation bases is between 0.001% and 10% by weight approximately relative to the total weight of the dye composition.

15. Composition according to Claim 12, in which the amount of each of the couplers is between 0.001% and 10% by weight approximately relative to the total weight of the dye composition.

16. Composition according to any one of Claims 11 to 15, comprising a cosmetically acceptable medium suitable for dyeing keratin fibres.

17. Composition according to any one of Claims 11 to 16, comprising an oxidizing agent.

18. Process for the oxidation dyeing of keratin fibres, **characterized in that** a dye composition as defined in any one of Claims 11 to 16 is applied to the fibres in the presence of an oxidizing agent, for a time that is sufficient to develop the desired coloration.

19. Process according to Claim 18, in which the oxidizing agent is chosen from hydrogen peroxide, urea peroxide, alkali metal bromates, persalts, peracids and oxidase enzymes.

20. Multi-compartment device in which a first compartment contains a dye composition as defined in any one of Claims 11 to 16 and a second compartment contains an oxidizing agent.

21. Use of the composition defined in Claims 11 to 17 for dyeing keratin fibres.

## Patentansprüche

1. *p*-Phenylendiaminderivate der Formel (I), die mit einer Pyrrolidinylgruppe substituiert sind, und ihre Additionssalze: 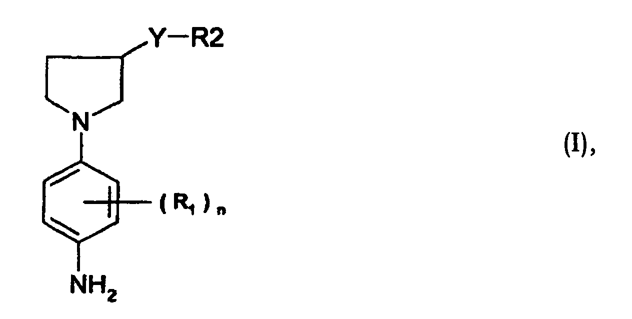 wobei in der Formel:
• n im Bereich von 0 bis 4 liegt, mit der Maßgabe, dass die Gruppen R₁ gleich oder verschieden sein können, wenn n 2 bedeutet oder darüber liegt,
• R₁ ein Halogenatom; eine gesättigte oder ungesättigte, aliphatische oder alicyclische C₁₋₆-Kohlenwasserstoffkette bedeutet, wobei ein oder mehrere Kohlenstoffatome der Kohlenwasserstoffkette durch ein oder mehrere Sauerstoffatome, Stickstoffatome, Siliciumatome oder Schwefelatome oder durch eine Gruppe SO oder SO₂ ersetzt sein können, wobei die Gruppe R₁ weder eine Peroxybindung noch eine Diazogruppe, Nitrogruppe oder Nitrosogruppe enthält; wobei die Kette mit einem oder mehreren Halogenatomen, einer oder mehreren Gruppen Hydroxy, Amino, Mono- oder Dialkyl(C₁₋₄)amino, Trialkyl(C₁₋₄)ammonium substituiert sein kann,
• Y eine kovalente Bindung ist oder eine geradkettige oder verzweigte C₁₋₁₄-Alkylenkette bedeutet, wobei ein oder mehrere Kohlenstoffatome dieser Kette durch ein Sauerstoffatom, Stickstoffatom, Schwefelatom, SO oder SO₂ ersetzt sein können; wobei die Kette mit einer Gruppe Hydroxy, C₁₋₆-Alkoxy, Amino, C₁₋₆-Alkylamino oder C₁₋₆-Dialkylamino substituiert sein kann; wobei die Kette eine oder mehrere Ketofunktionen tragen kann,
• R₂ einen Imidazolring bedeutet.

2. Derivate nach Anspruch 1, bei denen n 0 oder 1 beträgt.

3. Derivate nach Anspruch 1 oder 2, wobei R₁ ein Halogenatom, eine C₁₋₄-Alkylgruppe, eine C₁₋₄-Hydroxyalkylgruppe, eine C₁₋₄-Aminoalkylgruppe, eine C₁₋₄-Alkoxygruppe, eine C₁₋₄-Hydroxyalkoxygruppe bedeutet.

4. Derivate nach Anspruch 1 oder 3, wobei R₁ unter Methyl, Hydroxymethyl, 2-Hydroxyethyl, 1,2-Dihydroxyethyl, Methoxy, Isopropyloxy, 2-Hydroxyethoxy ausgewählt ist.

5. Derivate nach einem der Ansprüche 1 bis 4, wobei Y eine kovalente Bindung ist.

6. Derivate nach einem der Ansprüche 1 bis 5, wobei Y eine C₁₋₈-Alkylenkette ist, die eine oder mehrere Einheiten enthalten kann, die unter -O-, -NR'- oder -NR'CO- ausgewählt sind, wobei R' Wasserstoff oder C₁₋₄-Alkyl bedeutet.

7. Derivate nach einem der Ansprüche 1 bis 6, wobei Y -O-, -NR'-, -S-, -SO- oder -SO₂- bedeutet.

8. Derivate nach Anspruch 8 oder 9, wobei R₂ über eines der Stickstoffatome des Imidazolrings an Y gebunden ist.

9. Derivate nach einem der Ansprüche 1 bis 8, die unter 4-(3-Imidazol-1-yl-pyrrolidin-1-yl)-phenylamin, [1-(4-Aminophenyl)-pyrrolidin-3-yl]-(3-imidazol-1-yl-propyl)-amin, 2-Methyl-[1-(4-Aminophenyl)-pyrrolidin-3-yl]-(3-imidazol-1-yl-propyl)-amin, 4-(3-Imidazol-1-yl-pyrrolidin-1-yl)-2-methylphenylamin ausgewählt sind.

10. Derivate nach Anspruch 9, die unter 4-(3-Imidazol-1-yl-pyrrolidin-1-yl)-phenylamin, [1-(4-Aminophenyl)-pyrrolidin-3-yl]-(3-imidazol-1-yl-propyl)-amin, 2-Methyl-[1-(4-aminophenyl)-pyrrolidin-3-yl]-(3-imidazol-1-yl-propyl)-amin, 4-(3-Imidazol-1-yl-pyrrolidin-1-yl)-2-methylphenylamin ausgewählt sind.

11. Farbmittelzusammensetzung, die als Oxidationsbase mindestens ein *p*-Phenylendiaminderivat der Formel (I) enthält, wie es in einem der Ansprüche 1 bis 10 definiert ist.

12. Zusammensetzung nach Anspruch 11, die ferner einen Kuppler enthält, der unter den *m*-Phenylendiaminen, *m*-Aminophenolen, *m*-Dihydroxybenzolen, Naphthalinkupplern, heterocyclischen Kupplern und deren Additionssalzen ausgewählt ist.

13. Zusammensetzung nach einem der Ansprüche 11 oder 12, die eine zusätzliche Oxidationsbase enthält, die von den Oxidationsbasen der Formel (I) verschieden ist und unter den p-Phenylendiaminen, Bisphenylalkylendiaminen, p-Aminophenolen, o-Aminophenolen, heterocyclischen Basen und deren Additionssalzen ausgewählt ist.

14. Zusammensetzung nach einem der vorhergehenden Ansprüche 11 bis 13, wobei der Mengenanteil jeder Oxidationsbase im Bereich von etwa 0,001 bis 10 Gew.-% des Gesamtgewichts der Farbmittelzusammensetzung liegt.

15. Zusammensetzung nach Anspruch 12, wobei der Mengenanteil jedes Kupplers im Bereich von etwa 0,001 bis 10 Gew.-% des Gesamtgewichts der Farbmittelzusammensetzung liegt.

16. Zusammensetzung nach einem der Ansprüche 11 bis 15, die ein kosmetisch akzeptables Medium enthält, das zum Färben von Keratinfasern geeignet ist.

17. Zusammensetzung nach einem der Ansprüche 11 bis 16, die ein Oxidationsmittel enthält.

18. Verfahren zum oxidativen Färben von Keratinfasern, **dadurch gekennzeichnet, dass** auf die Fasern eine Farbmittelzusammensetzung nach einem der Ansprüche 11 bis 16 in Gegenwart eines Oxidationsmittels während einer Zeitspanne, die zur Bildung der gewünschten Färbung ausreichend ist, aufgebracht wird.

19. Verfahren nach Anspruch 18, wobei das Oxidationsmittel unter Wasserstoffperoxid, Harnstoffperoxid, Alkalimetallbromaten, Persalzen, Persäuren und Oxidasen ausgewählt ist.

20. Vorrichtung mit mehreren Abteilungen, wobei eine erste Abteilung eine Farbmittelzusammensetzung nach einem der Ansprüche 11 bis 16 und eine zweite Abteilung ein Oxidationsmittel enthält.

21. Verwendung der Zusammensetzung nach einem der Ansprüche 11 bis 17 zum Färben von Keratinfasern.
